(19) Europäisches Patentamt
European Patent Office
Office européen des brevets

(11) Publication number: 0 055 923
A1

# (12) EUROPEAN PATENT APPLICATION

(21) Application number: 81306113.2

(22) Date of filing: 23.12.81

(51) Int. Cl.³: C 12 Q 1/50

(30) Priority: 23.12.80 GB 8041316

(43) Date of publication of application: 14.07.82 Bulletin 82/28

(84) Designated Contracting States: BE CH DE FR IT LI NL

(71) Applicant: NATIONAL RESEARCH DEVELOPMENT CORPORATION
P.O. Box 236 Kingsgate House 66-74 Victoria Street
London SW1E 6SL(GB)

(72) Inventor: Chipperfield, Barbara
284 Cottingham Road
Hull North Humberside(GB)

(74) Representative: Percy, Richard Keith
National Research Development Corporation Patent Department P.O. Box 236 Kingsgate House 66/74
Victoria Street
London SW1E 6SL(GB)

## (54) Assay for creatine kinase isoenzymes.

(57) Two forms of creatine phosphokinase isoenzyme (CK) in animal tissue indicate damage to skeletal muscle (MM form) or cardiac damage (MB form). The invention provides a simpler and cheaper assay than used previously, arising from the discovery that certain metal ions inhibit MB form while inhibiting MM form less or even activating it. For human serum aluminium ions are preferred. From CK activity without the selective ion inhibitor total CK (MB + MM form) can be determined and a comparison of CK activity in the presence and absence of the selective ion inhibitor yields MB form activity. Creatine phosphate and ADP are normally added to determine CK activity.

EP 0 055 923 A1

Croydon Printing Company Ltd.

ASSAY FOR CREATINE KINASE ISOENZYMES

This invention relates to an assay for determination of creatine kinase (CK) isoenzymes.

Creatine kinase (CK), or creatine phosphokinase as it is more properly termed, is an enzyme which catalyses the transfer of phosphate from ATP to creatine, being associated with various animal tissues. In mammals CK exists as three different isoenzymes, each associated with a particular type of tissue in the body: the MM form (alternatively known as CK-3) associated with skeletal muscle and other tissues, the BB form (alternatively known as CK-1) mainly with brain tissue but also with stomach and intestinal tissue, and the MB hybrid form (alternatively known as CK-2) with heart tissue. Each such form of enzyme is a dimer composed of M and/or B sub-units.

In certain circumstances serum levels of CK isoenzymes become elevated, in particular when the associated tissues are damaged causing release of the corresponding isoenzymes, and increases in the serum concentrations of particular CK isoenzymes have been used to diagnose and confirm damage to the corresponding tissues. Thus a rise in the serum levels of the MM isoenzyme usually indicates damage to skeletal muscle. Increases in the serum levels of the MB isoenzyme confirm suspected cardiac damage after clinical observation of "heart attack" symptoms.

Hereinafter the generic term "creatine kinase isoenzyme" is abbreviated to CK, the three forms thereof merely to MM, BB and MB, and the sub-units thereof merely to M and B.

At present two main methods are used to determine the serum levels of CK: 1) a method which depends upon a slow laborious electrophoretic or chromatographic separation of the isoenzymes, and 2) a method which employs an antibody to MM as an inhibitor thereof. In this second method the CK activity of the serum is determined in the presence of the anti-MM antibody inhibitor, the residual activity being that due to B. It is assumed that the BB isoenzyme will not be present in serum unless brain damage has occurred. Therefore MB is determined by doubling the value found for B activity. This method is disadvantageous because the MM

antibody is difficult and costly to prepare. One commercial kit includes sodium azide, a dangerous reagent, as a storage preservative.

The present invention provides a method for determination of the concentration of MB form or MM form CK isoenzymes present in a sample of animal tissue, especially human serum, in which the CK activity of the sample is determined in the absence as well as the presence of a concentration of a metal ion which selectively inhibits the activity of the MB form of the enzyme in preference to that of the MM form of the enzyme, and in which the CK activities of the sample in the absence and presence of the inhibiting metal ion are compared.

Any metal ion which for tissue of a particular species of animal selectively inhibits the activity of MB in preference to MM can be used in the method of the invention at an inhibiting concentration and for assay of a sample taken from tissue of that species. The most preferred metal ion for use in assay of human serum is aluminium: it inhibits MB strongly while activating MM.

The effect of introducing a metal ion varies according to the species of animal from which the tissue is derived. Nevertheless, tests on rabbit isoenzymes are considered to be a reaonsable general guide to performance on human serum. The following ions were tested on MM and BB isoenzymes isolated from rabbits:-

divalent ions - calcium, strontium, barium, cobalt, nickel, copper, zinc, indium, tin and lead

trivalent ions - aluminium, scandium, titanium, chromium, manganese, iron, cobalt, ruthenium, rhodium, iridium, lanthanum, cerium, neodymium, dysprosium, erbium and ytterbium.

The ions which inhibited BB more strongly than MM were aluminium, ytterbium, iron, lanthanum, lead, iridium, dysprosium and erbium. Aluminium was the only ion to activate MM while inhibiting BB.

Magnesium ions are a well known additive in CK assays and are used in order to complex the ATP and assist in expressing CK activity. They are not, of course, usable as the selective ions

in the method of the invention, but are preferably added together with the aluminium or other selective ions.

The following description refers to assay of samples of human serum using aluminium as the selective ions. The invention is, of course, applicable to other kinds of sample of human or non-human animal tissue in which the presence of BB is not expected and other selective ions are usable mutatis mutandis.

The CK activity may be determined by any suitable method and means, including those previously used. Thus the CK activity may be determined by incubation of the sample together with a creatine substrate for a given period of time after which the extent of reaction is determined and the CK activity calculated. For example, the extent of the reaction:-

$$\text{Creatine phosphate} + \text{ADP} \xrightarrow{\text{CK isoenzyme}} \text{ATP} + \text{Creatine}$$

can be monitored colorimetrically. Thus creatine can be determined using a diacetyl/alpha-naphthol reagent to produce a pink coloration, or phosphate determined by the phosphomolybdate method. In principle, the creatine/creatine phosphate reaction may be monitored continuously. The well known hexokinase/glucose-6-phosphate dehydrogenase method, which produces NADPH, the appearance of which may be monitored by optical density measurements, is not suitable without modification, at least in the preferred embodiment of this invention, since aluminium ion inhibits hexokinase.

The concentration of the B present in the sample is estimated by comparison of the results obtained for CK activity in the presence and absence of the aluminium ions. The results obtained may be compared with results previously obtained from solutions having known concentrations of MM and MB. Preferably the conditions for assay of CK activity are chosen (as hereinafter explained) so that MB and therefore B activity is substantially completely inhibited and only M activity is measured in the presence of the aluminium ions. In this latter case the decrease in activity in the presence of the aluminium ions is a measure of the B of the

MB, the residual activity being due to the M of the MB and MM. As in prior assays, it is assumed that the sample does not contain any BB unless brain damage has occurred.

The aluminium cations can be used in association with any non-interfering anions and can be added, for example, as the nitrate, chloride or sulphate.

The selective effect of the aluminium ions depends upon their concentration in the final assay mixture (the CK reaction mixture). In human serum samples, MB is inhibited significantly and the MM form is activated at aluminium ion concentrations of above 2mM, especially above 2.5mM. At too high a concentration of aluminium ions, however, the MM form also becomes inhibited and a preferred upper limit is 5mM. Also, when the ADP concentration is relatively low to aluminium ion concentration, aluminium ions precipitate as the hydroxide. At 10mM ADP, the aluminium ion concentration is best not more than 4mM.

When, as is preferred, the assay is carried out in the presence of magnesium ions, the concentration thereof should be sufficient to inhibit MB significantly at the chosen aluminium ion concentration. When both aluminium and magnesium concentrations are low, the MB is insufficiently inhibited, but at low aluminium concentration the MB can be inhibited if the magnesium ion concentration is high enough. The range 6 to 15mM, preferably 8 to 15mM, of magnesium ion concentration in the final assay mixture is generally best.

The pH of the CK reaction mixture before adding the aluminiun ions is normally from 7.0 to 7.5, preferably from 7.1 to 7.4. At too low a pH (around 7.0) non-enzyme-catalysed reaction of creatine phosphate with ADP occurs to a significant extent. This means that blank assays performed for comparison have high background levels of CK activity, making changes in CK activity more difficult to detect. Inhibition of MB decreases with increasing pH: at too high a pH (around 7.5) the degree of inhibition of MB becomes too low. A pH of 7.2 to 7.4 is the most preferred range.

High blank values are also obtained if the concentration of creatine phosphate (CP) in the assay mixture is excessive. It is preferred to keep it at less than 14mM. However, the CK enzyme has only limited affinity for the CP substrate, expressed by the Michaelis constant $K_m$. It is only 2mM for human MB but 3.5mM for human MM. In order to use the assay method for human MM the lowest tolerable CP concentration in the CK reaction mixture (final assay mixture) is normally about 7mM and at least 8mM is preferred.

To keep the creatine phosphate concentration fairly low and thereby avoid high blanks, it is preferable to keep the concentration of ADP in the final assay mixture a little higher, for example at 10mM if the CP concentration is 8mM. Too high an ADP concentration is uneconomic and might cause undesirably high inhibition of CK. Generally it will be in the range 8 to 12mM depending on the concentration of aluminium ions and other factors.

The final assay mixture can contain other usual additives, for example a reagent for maintaining the creatine kinase isoenzyme in reduced form. A preferred such agent is a thiol group-containing agent, e.g. glutathione.

The assay can be carried out at any conventional temperature appropriate to expression of CK, preferably around 37°C when the creatine phosphate - ADP reaction is used to monitor CK.

Reagents for use in the method of the invention can be supplied in the form of multi-component kits. A kit can comprise (1) a substrate for CK, preferably creatine phosphate, and if necessary a co-reagent for reaction of the CK substrate to be catalysed by the CK, for example ADP when the substrate is creatine phosphate, and (2) the selective ions, preferably aluminium ions when human serum is to be assayed. The first component preferably comprises magnesium ion, creatine phosphate and ADP and optionally other compatible additives, e.g. glutathione. Buffer can be provided separately for making up a solution of the first component. Preferably the kit includes an inhibitor of CK activity and a colour-indicating agent. The colour-indicating agent can comprise

two or more reagents and preferably at least part of the colour-indicating agent is present as a third, separate component. For example, the diacetyl-alpha naphthol colour-indicating agent can be used. The preferred kit then contains the alkaline alpha-naphthol reagent and an inhibitor of (total) CK activity as a third component and diacetyl as a separate fourth component or as part of the first component. A suitable inhibitor of total CK activity is p-hydroxymercuribenzoate which can be added in the form of p-chloromercuribenzoate.

The proportions of magnesium ions, creatine phosphate and ADP in the preferred first component will conveniently be such that on addition of the aluminium ion component and after any necessary dilution, the final assay mixture contains the preferred proportions of the various reagents as set out above.

The invention is illustrated by the following Examples.

Example 1

The MB CK isoenzyme activity of human serum samples was determined as follows.

20-100 microlitres of serum were added to 2 ml of 0.1M Tris/acetate buffer (pH 7.4) containing 8mM $MgCl_2$, 10mM creatine phosphate, 3mM glutathione and 4mM ADP and incubated at 37°C for 20 minutes. At completion of incubation the CK reaction was stopped by addition of 0.2 ml, 0.05M p-chloromercuribenzoate (pCMB), 1 ml alpha-naphthol (20 mg/ml) in alkaline solution, 30 g NaOH, 80 g $Na_2CO_3$ in 500 ml of water. The pCMB solution was made by dissolving the solid in sodium hydroxide and adding dilute hydrochloric acid until the pCMB is only just in solution. 1 ml Diacetyl solution (0.05% w/v) and 6 ml distilled water were then added to each tube. The tubes were incubated at 37°C for 20 minutes and the resultant pink solution was measured colorimetrically using a Unicam SP 600 spectrophotometer, reading the extinction at 520 nm.

The above procedure was repeated for a sample of the same serum, but using 0.8 ml of 10mM aluminium nitrate in the reaction mixture in place of 0.8 ml of water when making up the 2 ml of

buffer solution. The concentration of aluminium ions in the reaction mixture was therefore about 4mM. Similarly the CK activity of the serum in the presence of the aluminium ions was determined. The two results obtained, one in the absence and the other in the presence of the aluminium ions, were compared, the difference between them indicating MB concentration in the serum.

This method was used to determine the MB present in the serum of 10 human patients, some of whom had been independently diagnosed as having suffered heart damage. The results are tabulated below. Total CK was determined independently by a standard method and MB was determined by the method of the invention (as percentage inhibition of total CK). The independently diagnosed condition for each patient is also reported.

| Patient | Day | MB (%age inhibition) | Total CK (μg/l) | Diagnosis |
|---|---|---|---|---|
| A | 1 | 0 | 213 | suspected myocardial infarction |
| A | 3 | 0 | 171 | |
| B | 2 | 0 | 216 | suspected myocardial infarction |
| B | 3 | 0 | 51 | |
| C | 1 | 0 | 283 | chest pains after cardiac catheter |
| D | 1 | 47 | 1610 | |
| D | 2 | 23 | 687 | myocardial infarction |
| D | 3 | 0 | 414 | |
| E | 1 | 25 | 1055 | query of myocardial infarction or pulmonary embolus |
| F | 1 | 27 | 224 | dermatomyocitis |

Continued

Continued

| Patient | Day | MB (%age inhibition) | Total CK ($\mu g/l$) | Diagnosis |
|---|---|---|---|---|
| G | 1 | 13 | 738 | myocardial infarction |
| H | 1 | 0 | 223 | cardiovascular accident |
| I | 1 | 46 | 1520 | |
| I | 2 | 0 | 579 | myocardial infarction |
| I | 3 | 0 | 172 | |
| J | 1 | 0 | 315 | pneumonia |
| K | 1 | 9 | 478 | myocardial infarction |

The results obtained were in complete agreement with the clinical diagnosis. Patient F was diagnosed as suffering from dermatomyocitis for which elevated MB levels were expected.

Example 2

A test kit for assay of human serum was prepared, having the following 3 separate components:-

| | | |
|---|---|---|
| Solution A: (total volume = 55 ml) | | 10 ml of a 80mM solution of magnesium acetate in buffer; 10 ml of a 30mM solution of glutathione in buffer; 10 ml of a 100mM solution of ADP in distilled water adjusted to pH 7.2; 10 ml of a 80mM solution of phosphocreatine (creatine phosphate) in buffer; 15 ml of additional buffer, which was a 0.1M aqueous solution of Tris acetate containing 0.1% w/v bovine serum albumin. |
| Solution B: | | 6g NaOH; 16g Sodium carbonate ($Na_2CO_3.10H_2O$) 2g Alpha-naphthol; 20 ml of a 0.05M solution of pCMB made up as in Example 1; Distilled water to make 100 ml. |

Solution C: 0.007% w/v Diacetyl in distilled water.

The method of assay was as follows. Up to 100 microlitres of human plasma, diluted as necessary with 0.9% w/v NaCl solution, was added to 1.1 ml of solution A, 0.8 ml of distilled water $H_2O$ (or 0.8 ml, 10mM aluminium nitrate) and the reaction mixture was incubated at 37°C for 20 minutes. The concentrations of the various components in the reaction mixture were therefore approximately 4mM aluminium ions, 8mM magnesium ions, 10mM ADP, 8mM creatine phosphate and 3mM glutathione. The CK reaction was stopped by the addition of 1 ml of solution B, and 7 ml of solution C. For colour development the mixture was incubated for 25 minutes at 37°C, and the pink coloration determined at 520 nm as in Example 1.

This method was used to determine MB as described in Example 1. Total CK was also determined from the activity of the sample in the absence of aluminium ions. A pH of 7.2 in the final assay mixture, before addition of aluminium ions, was used in Example 2.

CLAIMS

1. A method for the assay of the concentration of MB form or MM form CK (creatine phosphokinase isoenzyme) present in a sample of animal tissue, in which one of the forms is selectively inhibited in preference to the other and in which the CK activities of the sample in the absence and presence of the inhibition are compared, characterised in that the activities of the sample in the absence and presence of a concentration of metal ions which selectively inhibits the activity of MB form in preference to MM form are compared.

2. A method according to Claim 1 characterised in that the sample is of human serum and the selective ions are trivalent aluminium cations.

3. A method according to Claim 2 characterised in that the CK activities are compared in final assay mixtures in which the aluminium ion concentration is from 2 to 5 millimolar and in which aluminium ions are absent.

4. A method according to Claim 3 characterised in that the final assay mixtures contain magnesium.

5. A method according to Claim 4 characterised in that the magnesium ion concentration in the final assay mixtures is from 6 to 15 millimolar.

6. A method according to Claim 1, 2, 3, 4 or 5 characterised in that the CK activites are compared by adding creatine phosphate and adenosine diphosphate (ADP) to the sample to carry out a reaction thereof catalysed by CK present in the sample and the resultant final assay mixtures on which the comparison is made have a pH of from 7.0 to 7.5 in the absence of the selective ions.

7. A method according to Claim 6 characterised in that the pH is from 7.2 to 7.4.

8. A method according to Claim 6 or 7 characterised in that the sample is of human serum and the concentration of creatine phosphate in the final assay mixture is from 8 to 14 millimolar.

9. A method according to Claim 6, 7 or 8 characterised in that the concentration of ADP in the final assay mixture exceeds that of creatine phosphate.

10. A method according to Claim 6, 7, 8 or 9 characterised in that the concentration of ADP in the final assay mixture is from 8 to 12 millimolar.

11. A method according to Claim 6, 7, 8, 9 or 10 characterised in that the CK activity is compared colorimetrically by including a colour-indicating agent in the final assay mixture.

12. A multi-component kit for use in a method of assay of the concentration of MB form or MM form CK present in animal tissue, in which a first component comprises a substrate for CK effective to produce a reaction catalysed by CK, and a second, separate component comprises a reagent or reagents for inhibiting one of the said forms in preference to the other, characterised in that the second component of the kit comprises metal ions which are capable of selectively inhibiting the MB form in preference to the MM form.

13. A kit according to Claim 12 characterised in that the selective ions are of aluminium.

14. A kit according to Claim 12 or 13 characterised in that the first component comprises magnesium ions, creatine phosphate and ADP and the kit also includes a colour-indicating agent, at least part of which is present as a third, separate component.

15. A kit according to Claim 14 characterised in that the first component includes also a thiol group-containing reagent, the second component comprises sodium hydroxide, sodium carbonate, alpha-naphthol and an inhibitor of total CK activity and the third component comprises diacetyl.

0055923

European Patent Office

# EUROPEAN SEARCH REPORT

Application number

EP 81 30 6113

## DOCUMENTS CONSIDERED TO BE RELEVANT

| Category | Citation of document with indication, where appropriate, of relevant passages | Relevant to claim |
|---|---|---|
| Y | CLINICAL CHEMISTRY, vol.25, no.3, 1979<br>G. SZASZ et al.:"Creatine Kinase in Serum:6. Inhibition by Endogenous Polyvalent Cations, and Effect of Chelators on the Activity and Stability of Some Assay Components"<br>pages 446-452<br>* pages 446-452 | 1 |
| Y | CLINICAL CHEMISTRY, vol.26, no.8, 1980<br>D.A. NEALON et al.:"Effect on Cations on the Human Creatine Kinase Isoenzymes"<br>pages 1137-1139<br>* pages 1137-1139 * | 1 |
| A | CHEMICAL ABSTRACTS, vol.92, no.1, January 7, 1980, page 233, abstract no.2300w<br>COLUMBUS, Ohio (US)<br>& Proc. Natl. Acad. Sci. U.S.A. 1979, 76(10), 5080-4<br>F.C. WOMACK et al.:"Proton-dependent Inhibition of Yeast and Brain Hexokinases by Aluminum in ATP Preparations"<br>* the whole abstract * | 2,4-7 |

--

./..

CLASSIFICATION OF THE APPLICATION (Int. Cl. 3)

C 12 Q 1/50

TECHNICAL FIELDS SEARCHED (Int.Cl. 3)

C 12 Q
G 01 N
C 12 N

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 26-03-1982 | DE LUCA |

EPO Form 1503.1 06.78

European Patent Office

# EUROPEAN SEARCH REPORT

Application number
EP 81 30 6113



| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| Y | CHEMICAL ABSTRACTS, vol.92, no.11, March 17, 1980, page 220, abstract no.89945g COLUMBUS, Ohio (US) & Methods Enzymol. 1979, 63 (Enzyme Kinet. Mech., Part A), 437-67 J.W. WILLIAMS et al.:"The Kinetics of Reversible Tight-Binding Inhibition" * the whole abstracts * -- | 1 | |
| | | | TECHNICAL FIELDS SEARCHED (Int. Cl.3) |
| A | BIOCHEMICAL JOURNAL, vol.57, 1954, A.H. ENNOR et al.:"Some Properties of Creatine Phospho-kinase" pages 203-212 * pages 203-212 * -- | | |
| Y | GB - A - 1 189 825 (SIGMA CHEMICAL) * the whole document * -- | 1,12, 14-15 | |
| Y | H.U. BERGMEYER:"METHODEN DER ENZYMATISCHEN ANALYSE", vol.1, 1974, Verlag Chemie WEINHEIM (DE) A.L. SIEGEL et al.:"Bestimmung mit Analysenautomaten" pages 821-825 * the whole article * -- ./.. | 1,12, 14-15 | |

0055923

European Patent Office

# EUROPEAN SEARCH REPORT

Application number

EP 81 30 6113

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| Y | CLINICAL CHEMISTRY, vol.25, no.3, 1979<br>G. SZASZ et al.:"Creatine Kinase in Serum:6. Inhibition by Endogenous Polyvalent Cations, and Effect of Chelators on the Activity and Stability of Some Assay Components"<br>pages 446-452<br>* pages 446-452 | 1 | C 12 Q 1/50 |
| | -- | | TECHNICAL FIELDS SEARCHED (Int.Cl. 3) |
| Y | CLINICAL CHEMISTRY, vol.26, no.8, 1980<br>D.A. NEALON et al.:"Effect on Cations on the Human Creatine Kinase Isoenzymes"<br>pages 1137-1139<br>* pages 1137-1139 * | 1 | C 12 Q<br>G 01 N<br>C 12 N |
| | -- | | |
| A | CHEMICAL ABSTRACTS, vol.92, no.1, January 7, 1980, page 233, abstract no.2300w<br>COLUMBUS, Ohio (US)<br>& Proc. Natl. Acad. Sci. U.S.A. 1979, 76(10), 5080-4<br>F.C. WOMACK et al.:"Proton-dependent Inhibition of Yeast and Brain Hexokinases by Aluminum in ATP Preparations"<br>* the whole abstract * | 2,4-7 | |
| | -- ./.. | | |

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document

X The present search report has been drawn up for all claims

| Place of search | Date of completion of the search | Examiner |
|---|---|---|
| The Hague | 26-03-1982 | DE LUCA |

EPO Form 1503.1 06.78

European Patent Office

# EUROPEAN SEARCH REPORT

Application number
EP 81 30 6113



| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| Y | CHEMICAL ABSTRACTS, vol.92, no.11, March 17, 1980, page 220, abstract no.89945g COLUMBUS, Ohio (US) & Methods Enzymol. 1979, 63 (Enzyme Kinet. Mech., Part A), 437-67 J.W. WILLIAMS et al.:"The Kinetics of Reversible Tight-Binding Inhibition" | | |
| | * the whole abstracts * | 1 | |
| | -- | | TECHNICAL FIELDS SEARCHED (Int. Cl.3) |
| A | BIOCHEMICAL JOURNAL, vol.57, 1954, A.H. ENNOR et al.:"Some Properties of Creatine Phospho-kinase" pages 203-212 | | |
| | * pages 203-212 * | | |
| | -- | | |
| Y | GB - A - 1 189 825 (SIGMA CHEMICAL) | | |
| | * the whole document * | 1,12, 14-15 | |
| | -- | | |
| Y | H.U. BERGMEYER:"METHODEN DER ENZYMATISCHEN ANALYSE", vol.1, 1974, Verlag Chemie WEINHEIM (DE) A.L. SIEGEL et al.:"Bestimmung mit Analysenautomaten" pages 821-825 | | |
| | * the whole article * | 1,12, 14-15 | |
| | -- ./.. | | |

European Patent Office

# EUROPEAN SEARCH REPORT

Application number
EP 81 30 6113



| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| A | BIOCHEMISTRY, vol.19, April 1, 1980, American Chemical Society, D. DUNAWAY-MARIANO et al.:"Investigations of Substrate Specificity and Reaction Mechanism of Several Kinases Using Chromium (III) Adenosine 5'-Triphosphate and Chromium (III) Adenosine 5'-Diphosphate" pages 1506-1515<br>* pages 1511-1515<br>----- | <br>1 | |
| | | | TECHNICAL FIELDS SEARCHED (Int. Cl.3) |